# EUROPEAN PATENT SPECIFICATION

(11) **EP 1 423 096 B1**
(45) Date of publication and mention of the grant of the patent: **16.08.2006**
(21) Application number: 02801623.6
(22) Date of filing: 27.08.2002
(51) Int. Cl.: A61K 9/16, A61K 9/51, B01D 9/00

(54) **A PROCESS FOR PREPARING CRYSTALLINE DRUG PARTICLES BY MEANS OF PRECIPITATION**
VERFAHREN ZUR HERSTELLUNG KRISTALLINER ARZNEIMITTELTEILCHEN DURCH AUSFÄLLUNG
PROCEDE DE PREPARATION DE PARTICULES CRISTALLINES DE MEDICAMENTS PAR PRECIPITATION

(30) Priority: 29.08.2001 US 315560 P
(43) Date of publication of application: 02.06.2004
(73) Proprietor: DOW GLOBAL TECHNOLOGIES INC., Midland, Michigan 48674 (US)
(72) Inventor: HITT, James, E., Midland, MI 48642 (US); TUCKER, Christopher, J., Midland, MI 48640 (US); EVANS, Jonathan, C., Midland, MI 48642 (US); CURTIS, Cathy, A., Midland, MI 48642 (US); SVENSON, Sonke, Midland, MI 48642 (US)
(74) Representative: Weickmann, Heinrich
(86) International application number: PCT/US2002/027444
(87) International publication number: WO 2003/032951

(56) References cited:
- WO-A-00/38811
- WO-A-00/53282
- WO-A-01/14036
- WO-A-01/32125
- WO-A-02/00198
- WO-A-02/00200
- US-A- 4 826 689
- US-B1- 6 221 398

## Description

The present invention relates to methods for the preparation of drug particles. More particularly, the present invention relates to the preparation of drug particles utilizing a continuous solvent precipitation method.

High bioavailability and short dissolution times are desirable attributes of a pharmaceutical end product. Bioavailability is a term meaning the degree to which a pharmaceutical product, or drug, becomes available to the target tissue after being administered to the body. Poor bioavailability is a significant problem encountered in the development of pharmaceutical compositions, particularly those containing an active ingredient that is poorly soluble in water. Poorly water soluble drugs tend to be eliminated from the gastrointestinal tract before being absorbed into the circulation.

It is known that the rate of dissolution of a particulate drug can increase with increasing surface area, such as by decreasing particle size. Furthermore, crystalline drug particles are desirable because of the greater stability as opposed to amorphous particles. Efforts have been made to control the size and morphology of drug particles in pharmaceutical compositions. The most commonly employed techniques involve precipitation of the drug substance. U.S. Patent 5,716,642 teaches the use of an acid-base precipitation method. However, the method described in the '642 patent results in a large concentration of salt which must be removed via dialysis in order to obtain relatively pure drug particles.

Solvent precipitation methods are described in U.S. Patent Nos. 4,826,689 and 6,221,398 B1, in Hasegawa et al, "Supersaturation Mechanism of Drugs from Solid Dispersions with Enteric Coating Agents, Chem. Pharm. Bull. Vol. 36, No. 12, p. 4941(1988), and Frederic Ruch and Egon Matijevic, Preparation of Micrometer Size Budesonide Particles by Precipitation, Journal of Colloid and Interface Science, 229, 207-211 (2000). In the standard method described in these references, a supersaturated solution of the compound to be crystallized is contacted with an appropriate 'anti-solvent' in a stirred vessel. Within the stirred vessel, the anti-solvent initiates primary nucleation which leads to crystal formation. However, the crystals that are formed are relatively large, whereas the smaller particles described by these references are amorphous. For the relatively large crystalline particles, these methods almost always require a post-crystallization milling step in order to increase particle surface area and thereby improve their bioavailability. However, milling has drawbacks, including yield loss, noise and dust. Even wet milling techniques, as described in as described in U.S. Patent No. 5,145,684, exhibit problems associated with contamination from the grinding media. Moreover, exposing a drug substance to excessive mechanical shear or exceedingly high temperatures can cause the drug to lose its activity.

The precipitation methods described in the above-cited prior art also have the disadvantage of being operated in a batch fashion. Batch can be difficult to scale up to a commercial operation and are generally more inefficient than continuous processes. Moreover, a continuous process will often result in a more uniform product than a batch process.

U.S. Patent No. 5,314,506 describes a continuous crystallization method which utilizes impinging jets to mix two streams together in order to precipitate crystalline particles. The resulting particles are crystalline, but are relatively large. Moreover, the method of the '506 patent relies upon the momentum of the two streams to mix the two streams together, which can make operation on a large scale difficult. The '506 patent also results in mixing of the entire quantity of the two streams all at once rather than gradual addition of one stream to the other, which can be undesirable.

It would be an advantage in the art of direct preparation of crystalline drug particles to provide a method which is continuous and allows for scalability, which is applicable to a wide breadth of drug substances and which does not require the use of subsequent milling of the drug particles.

In one aspect, the present invention is a process for preparing crystalline particles of a drug substance comprising recirculating an anti-solvent through a mixing zone, dissolving the drug substance in a solvent to form a solution, adding the solution to the mixing zone to form a particle slurry in the anti-solvent, and recirculating at least a portion of the particle slurry back through the mixing zone.

The present invention has the advantage of being a continuous process, resulting in a more efficient process and a more uniform product. The present invention has the additional advantage of having the ability to operate at a relatively low solvent ratio and increasing the drug level in the particle slurry, thereby increasing the drug to excipient ratio.

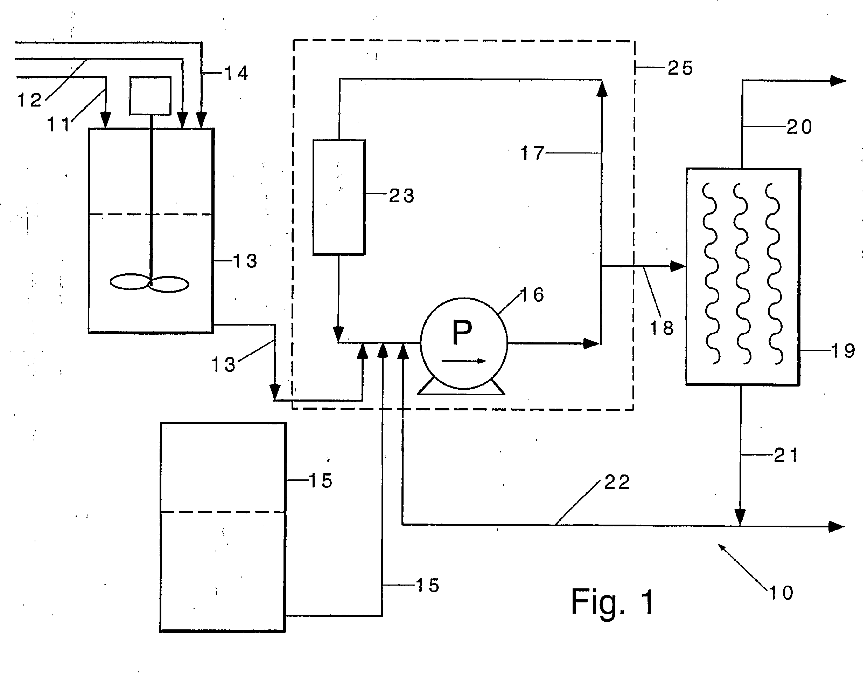

FIG. 1 is a schematic diagram illustrating the process of the present invention.

Figure 1 is a schematic diagram illustrating one embodiment of the continuous process 10 of the present invention. A drug substance 11 is mixed with an organic solvent 12 to form a solution 13.

The drug substance 11 which can be used in the process of the present invention can be any poorly water soluble drug. Suitable drug substances can be selected from a variety of known classes of drugs including, for example, analgesics, anti-inflammatory agents, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytic sedatives (hypnotics and neuroleptics), astringents, beta-adrenoceptor blocking agents, blood products and substitutes, cardiacinotropic agents, contrast media, corticosterioids, cough suppressants (expectorants and mucolytics), diagnostic agents, diagnostic imaging agents, diuretics, dopaminergics (antiparkinsonian agents), haemostatics, immuriological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasidilators and xanthines. Preferred drug substances include those intended for oral administration. A description of these classes of drugs and a listing of species within each class can be found in Martindale, The Extra Pharmacopoeia, Twenty-ninth Edition, The Pharmaceutical Press, London, 1989.

The organic solvent 12 into which the drug is dissolved can be any organic solvent which dissolves the drug adequately. Generally, the higher the solubility of the drug in the solvent, the more efficient the process will be. The solvent should be miscible in anti-solvent. Preferably, the selected solvent exhibits ideal mixing behavior with anti-solvent so that the solution can be instantaneously distributed throughout the particle slurry, as described hereinbelow. Suitable organic solvents include but are not limited to methanol, ethanol, isopropanol, 1-butanol, trifluoroethanol, polyhydric alcohols such as propylene glycol, PEG 400, and 1,3-propanediol, amides such as n-methyl pyrrolidone, n,n-dimethylformamide, tetrahydrofuran, propionaldehyde, acetone, n-propylamine, isopropylamine, ethylene diamine, acetonitrile, methyl ethyl ketone, acetic acid, formic acid, dimethylsulfoxide, 1,3-dioxolane, hexafluoroisopropanol, and combinations thereof.

The concentration of drug in the solution is preferably as close as practical to the solubility limit of the solvent. Such concentration will depend upon the selected drug and solvent but is typically in the range of from 0.1 to 20.0 weight percent.

Optionally, one or more stabilizers 14 can be introduced into the solution 13. Stabilization is defined herein to mean that the resulting drug particles do not grow substantially, such that particles prepared from precipitation in the presence of stabilizer are generally smaller than those prepared without a stabilizer. Stabilization should be carried out in such a way that addition of additional drug solution substantially results in new particle formation and not growth of existing particles.

The choice of stabilizer or stabilizers will depend upon the drug molecule. Generally, polymeric stabilizers are preferred. Examples of particle stabilizers include phospholipids, surfactants, polymeric surfactants, vesicles, polymers, including copolymers and homopolymers and biopolymers, and/or dispersion aids. Suitable surfactants include gelatin, casein, lecithin, phosphatides, gum acacia, cholesterol, tragacanth, fatty acids and fatty acid salts, benzalkonium chloride, glycerol mono and di fatty acid esters and ethers, cetostearyl alcohol, cetomacrogol 1000, polyoxyethylene castor oil derivatives, polyoxyethylene sorbitan fatty acid esters, for example, the commercially available Tweens, polyethylene glycols, poly(ethylene oxide/propylene oxide) copolymers, for example, the commercially available Poloxomers or Pluronics, polyoxyethylene fatty acid ethers, for example, the commercially available Brijs, polyoxyethylene fatty acid esters, sorbitan fatty acid esters, for example, the commercially available Spans, colloidal silicon dioxide, phosphates, sodium dodecylsulfate, carboxymethylcellulose calcium, carboxymethylcellulose sodium, methylcellulose, hydroxyethylcellulose, hydroxypropylcellulose, hydroxypropylmethylcellulose, noncrystalline cellulose, magnesium aluminum silicate, triethanolamine, polyvinyl alcohol (PVA), sodium lauryl sulfate, polyvinylpyrrolidone (PVP), poly(acrylic acid), and other anionic, cationic, zwitterionc and nonionic surfactants. Other suitable stabilizers are described in detail in the Handbook of Pharmaceutical Excipients, published jointly by the American Pharmaceutical Association and The Pharmaceutical Society of Great Britain, the Pharmaceutical Press, 1986. Such stabilizers are commercially available and/or can be prepared by techniques known in the art.

An important part of the continuous process of the present invention is the use of a mixing zone 25. Utilization of the mixing zone results in high velocity, high turbulence, efficient heat exchange in a form that is easily scalable. In the embodiment shown in Figure 1, mixing zone 25 comprises pump 16 together with recirculation loop 17.

Anti-solvent 15 is pumped by way of pump 16 through the recirculation loop 17. The term "anti-solvent" is defined as any material in which the drug is poorly soluble, defined as meaning less than 10 mg/ml. Water is the preferred anti-solvent. In one embodiment, the anti-solvent contains one or more stabilizers, such as those stabilizers described above.

The solution 13 is then added to the anti-solvent in the mixing zone 25 to form a slurry of drug particles in anti-solvent, referred to herein as a particle slurry. As the solution is added to the anti-solvent in the mixing zone, the resulting particle slurry is mixed. Any external device which imparts intense mixing of the particle slurry in the mixing zone 25 can be used, as long as the selected device will permit continuous operation. "Intense mixing" is defined herein as meaning that a uniformly supersaturated particle slurry is formed prior to new particle nucleation. The mixing should be sufficiently intense so as to result in nearly instantaneous dispersion of the solution across the particle slurry before new particle growth occurs. As with stabilization, mixing should be carried out in such a way that addition of additional drug solution substantially results in new particle formation and does not substantially result in growth of existing particles. Such intense mixing results in supersaturation of the drug substance in the slurry, causing drug particles to precipitate into small particles having a crystalline structure. If stabilization fails, growth on existing particles predominates over new particle formation, resulting in large crystals which may require milling to meet bioavailability requirements.

Advantageously, steady-state conditions can be approached gradually using the process of the present invention, rather than all at once. In the embodiment shown in Figure 1, which contains loop 17 in combination with pump 16, preferably, the combination of steady-state flow rate, anti-solvent fluid properties, and line diameter in loop 17 are sufficient to achieve a Reynolds number of at least 2500, more preferably at least 5000, even more preferably at least 10,000 in the loop 17. The drug solution can be added to the mixing zone slowly or quickly as desired. The rate of addition of drug solution is not critical, so long as the relative flow rates of the solution and the particle slurry are sufficient to create intense mixing. For example, for the embodiment shown in Figure 1, the rate of addition of the drug solution can be about (0.6 x V)/minute wherein V is the volume of the mixing zone.

Examples of devices which may be used to mix the two streams in the mixing zone include one or more of a centrifugal pump, an in-line homogenizer, an ultrasonic mixer, an atomizer, and a colloid mill. Combinations of such mixing devices may also be used, especially in those cases where it is desirable to increase residence time in the mixing zone. In the embodiment illustrated in Figure 1, centrifugal pump 16 together with a recirculation loop 17 serves as a mixing device.

The particle slurry will contain new drug particles that are continuously being formed by precipitation, as well as existing drug particles that have previously been formed and recirculated and have been stabilized to substantially prevent further growth. Desirably, the concentration of drug in the particle slurry can gradually increase as steady-state conditions are approached. Once steady-state is reached, it is desirable to have the drug concentration as high as is practical. A high drug concentration is an advantage of the present invention, because with a high drug concentration, the quantity of stabilizer is efficiently utilized, leading to a relatively low quantity of stabilizer relative to the drug. Preferably, the drug concentration is at least 0.01 weight percent, more preferably at least 0.1 weight percent and even more preferably at least 0.5 weight percent at equilibrium

Optionally, one or more stabilizers may be added to the anti-solvent. Suitable particle stabilizers include those listed above for inclusion in the solution. The particular stabilizer or stabilizers selected for use in the anti-solvent can be the same or can be different from the stabilizer(s) in the solution. The weight ratio of drug to total stabilizer in the particle slurry is from 0.1:1 to 10:1.

Advantageously, additional excipients can be added to either the solution or to the anti-solvent, either before or after the drug particles are formed, in order to enable the drug particles to be homogeneously admixed for appropriate administration. Suitable excipients include polymers, absorption enhancers, solubility enhancing agents, dissolution rate enhancing agents, bioadhesive agents, and controlled release agents. More particularly, suitable excipients include cellulose ethers, acrylic acid polymers, and bile salts. Other suitable excipients are described in detail in the Handbook of Pharmaceutical Excipients, published jointly by the American Pharmaceutical Association and The Pharmaceutical Society of Great Britain, the Pharmaceutical Press, 1986. Such excipients are commercially available and/or can be prepared by techniques known in the art.

In a preferred embodiment, the particle slurry is recycled back through the mixing zone. The particle slurry in the mixing zone is controlled at a reduced temperature by way of heat exchanger 23. Preferably, the temperature of the particle sluny in the mixing zone is controlled at less than 65 °C, more preferably less than 30 °C, even more preferably less than 23 °C, and most preferably less than 10 °C. The lower limit of the temperature of the particle slurry is the freezing point of the anti-solvent, or 0 °C if the anti-solvent is water. Temperatures which are too high could lead to undesirable particle growth.

Once the particle slurry has recirculated at reduced temperature and the solution has been introduced, equilibrium conditions can be achieved. In a preferred embodiment, anti-solvent feed line 15 will act as a anti-solvent make-up line to make up for any anti-solvent lost in the process.

An optional slip stream 18 continuously permits at least a portion of the particle slurry to be fed to solvent removal step. Any solvent removal operation can be used, including membrane filtration, diafiltration and evaporation. In the embodiment illustrated in Figure 1, an evaporator 19 is shown. Any appropriate evaporator can be used, as long as it permits continuous operation and evaporates a substantial quantity of solvent 20, leaving drug particles suspended in anti-solvent, referred to herein as a stripped slurry 21. Examples of evaporators include a falling film evaporator and a wiped film evaporator. A wiped film evaporator is preferred, because such an evaporator helps to reduce any foaming that might occur during processing. The wiped film evaporator can be arranged either horizontally or vertically. The operating conditions of the evaporator will depend upon the solvent used. Preferably, the evaporator is held under vacuum and is operated at a temperature at least as high as the boiling point of the solvent.

In a preferred embodiment, the process of the present invention includes the step of passing at least a portion 22 of the stripped slurry back through the mixing zone 25. Advantageously, this step results in a higher drug particle concentration in the recirculated particle slurry and a lower solvent concentration, which in turn results in more efficient drug particle recovery and a higher drug to stabilizer ratio. Additionally, a lower solvent concentration results in generally lower particle size because solvent is not as available to facilitate drug migration and particle growth.

The resulting drug particles that are present in the stripped slurry are formed directly, without the need for subsequent milling. The drug particles in the stripped slurry preferably have a mean volume average particle size, without filtration, of less than 5 microns, more preferably less than 2 microns, and even more preferably less than 1 micron. The resulting drug particles are substantially crystalline in nature.

The process of the present invention desirably further comprises the step of recovering the drug particles. In one embodiment, recovering the drug particles comprises removing the anti-solvent from the particles. The anti-solvent can be removed directly after the particle slurry is formed, or the anti-solvent can be removed after any residual solvent is evaporated from the particle slurry. The choice will depend upon the concentration of solvent in the particle slurry and the chosen method to remove the anti-solvent. Removing the anti-solvent can be performed using any desirable means, including spray drying, spray freezing, gellation, (defined as gelling the particles with a polymer), lyophilization, or filtration.

The resulting drug particles are desirably redispersible in the anti-solvent with nearly the same particle size as the particles in the stripped slurry. Preferably, the mean particle size in the redispersed drug particles is within 60 percent of the particle size in the stripped slurry, more preferably within 50 percent, even more preferably within 30 percent, and yet even more preferably within 20 percent.

The following examples are for illustrative purposes only and are not intended to limit the scope of the claimed invention. Percentages are in weight percents unless otherwise stated.

### Examples

### Examples 1 through 3

A continuous precipitation process shown in Figure 1 was used. 150 grams of deionized water was recirculated using centrifugal pump (Cole- Parmer Model 75225-10) at maximum pump speed through recirculation loop 17 and through heat exchanger 23 (Exergy Inc. Model 00283-01, 23 series heat exchanger) until the temperature reached 5 °C. 30.8 grams of a solution of 5 wt percent Danazol and 2.5 wt percent Pluronic F-127 in methanol was added into the water over about 25 seconds. A particle slurry was formed. The particle size of the particle slurry was measured, without filtration, using a Coulter LS 230 and is listed in Table I below. The particle slurry was then fed to a wiped film evaporator having a jacket temperature of 40 °C, an absolute pressure of 10.5 mm Hg, and a feed rate of 10 mL/min. The particle size of the stripped slurry was measured, without filtration, using a Coulter LS 230 and is listed in Table I below. The stripped slurry was then fed back to the recirculation loop, with sufficient water being used to bring the total to about 150 grams. This precipitation procedure was repeated two more times using the amounts of materials listed in Table I, each repetition corresponding to examples 2 and 3, respectively.

### Example 4

After the third pass through the recirculation loop and the wiped film evaporator, the stripped slurry was sent back through the wiped film evaporator for a second pass. The wiped film evaporator had a jacket temperature of 40 °C, an absolute pressure of 10.5 mm Hg and a feed rate of 10 mL/min. The final slurry weight and particle size are listed in Table I.

**Table I**

| Example | Deionized Water (grams) | Danazol Solution (grams) | Final weight after evaporation (grams) | Mean volume average particle size (µm) | |
|---|---|---|---|---|---|
| | | | | Before evaporation | After evaporation |
| 1 | 150.0 | 30.8 | 96.7 | 0.156 | -- |
| 2 | 65.9 | 30.2 | 94.6 | 0.219 | 0.242 |
| 3 | 59.5 | 30.9 | 98.2 | 0.277 | 0.307 |
| 4 | 0 | 0 | 60.4 | 0.307 | 0.322 |

### Example 5

The stripped slurry from Example 4 was freeze dried 48 hours in a VirTis freeze dryer (catalog number 6201 3150) with an Edwards vacuum pump operated at maximum vacuum to isolate the drug particles. The drug particles were reconstituted by mixing with deionized water to a level of 1-2 wt percent solids and shaking by hand. The mean volume average particle size of the reconstituted freeze dried drug particles was 0.489 um, as measured, without filtration, using a Coulter LS 230.

### Examples 6-8

A continuous process shown in Figure 1 was used, except that the stabilizer was added to the anti-solvent rather than to the solution. 150.1 grams of deionized water containing 3.0 wt percent polyvinylpyrrolidone (55,000 molecular weight, Aldrich) was recirculated using a centrifugal pump (Cole- Parmer Model 75225-10) at maximum pump speed through recirculation loop 17 and heat exchanger 23 (Exergy Inc. Model 00283-01, 23 series heat exchanger) until the temperature reached 3 °C. 29.92 grams of a solution of 6.67 wt percent Naproxen in methanol was added into the water over about 25 seconds to form a particle slurry. A sample of the particle slurry was taken, and the particle size of the sample was measured, without filtration, using a Coulter LS 230. A portion of the particle slurry was removed (15-20 percent). The amount of particle slurry recycled from the previous example is listed in Table II. Additional Naproxen in methanol solution was then added into the particle slurry over about 25 seconds. This was repeated once more. Table II lists the amount of Naproxen solution added and the resulting particle sizes for all three of Examples 6-8.

**Table II**

| Example | Deionized Water/PVP (grams) | Naproxen Solution (grams) | Particle slurry recycled from previous example (grams) | percent Naproxen of total solids | Mean volume average particle size (µm) |
|---|---|---|---|---|---|
| 6 | 150.1 | 29.92 | -- | 30.7 | 0.230 |
| 7 | 0 | 25.12 | 150.44 | 47.0 | 0.373 |
| 8 | 0 | 20.83 | 145.68 | 57.1 | 0.414 |

### Example 9-11

A continuous process shown in Figure 1 was used, except that the stabilizer was added to the anti-solvent rather than to the solution. These examples demonstrated recycling at least a portion 22 of the stripped slurry back through recirculation loop 17. 150.39 grams of deionized water containing 3.0 wt percent polyvinylpyrrolidone (55,000 molecular weight, Aldrich) was recirculated using a centrifugal pump (Cole- Parmer Model 75225-10) at maximum pump speed through recirculation loop 17 and heat exchanger 23 (Exergy Inc. Model 00283-01, 23 series heat exchanger) until the temperature reached 3 °C. 30.10 grams of a solution of 6.67 wt percent Naproxen in methanol was added into the water over about 25 seconds to form a particle slurry. The particle slurry was then fed to a wiped film evaporator operating at 22-25 mm Hg absolute pressure and 30-40 °C jacket temperature to strip most of the solvent. Sufficient water was added to the stripped slurry to bring the total sluny to about 150 grams, and the entire quantity was then fed back through the recirculation loop 17. Additional Naproxen/methanol solution was then added to the recirculation loop, and the slurry was then sampled for particle size. The particle size of the stripped slurry was measured, without filtration, using a Coulter LS230. Table III lists the amount of water added to the slurry, the Naproxen solution added, and the resulting particle sizes for all three of Examples 9-11.

**Table III**

| Example | Deionized Water/PVP (grams) | Naproxen Solution (grams) | Stripped slurry from previous example (grams) | Percent Naproxen of total solids | Mean volume average particle size (µm) |
|---|---|---|---|---|---|
| 9 | 150.39 | 30.10 | -- | 30.8 | -- |
| 10 | 13.95* | 30.13 | 137.24 | 47.1 | 0.304 |
| 11 | 27.36* | 30.01 | 128.85 | 57.1 | 0.299 |

| | | | | | |
|---|---|---|---|---|---|
| * Deionized water containing no PVP. | | | | | |

### Examples 12 through 17

A continuous precipitation process shown in Figure 1 was used. 150.14 grams of deionized water containing 2.5 wt percent polyvinylpyrolidone (5 5,000 molecular weight, Aldrich) was recirculated using centrifugal pump (Cole- Parmer Model 75225-10) at maximum pump speed through recirculation loop 17 and through heat exchanger 23 (Exergy Inc. Model 00283-01, 23 series heat exchanger) until the temperature reached 3-4 °C. 30.06 grams of a solution of 7 wt percent Naproxen in methanol solution was added into the water over about 25 seconds to form a particle slurry. The particle slurry was then fed to a wiped film evaporator having a jacket temperature of 26-28 °C, an absolute pressure of 5-6 mm Hg, and a feed rate of about 15 mL/min. The particle size of the stripped slurry was measured, without filtration, using a Coulter LS 230 and is listed in Table IV below. Half of the stripped slurry was collected for isolation and the other half was then fed back to the recirculation loop, with sufficient water being used to bring the total to about 75 grams. About 75 grams of deionized water containing 2.5 wt percent polyvinylpyrolidone (55,000 molecular weight, Aldrich) was added to the recirculation loop to make up for the polymer collected in the isolation stream. This precipitation procedure was repeated five more times using the amounts of materials listed in Table IV, each repetition corresponding to examples 13 through 17, respectively.

**Table IV**

| Example | 2.5 wt percent PVP in water (grams) | Deionized Water (grams) | Recycled particle slurry (grams) | Naproxen Solution (grams) | Final weight after evaporation (grams) | Isolation particle slurry (grams) | Mean volume average particle size after evaporation (µm) |
|---|---|---|---|---|---|---|---|
| 12 | 150.14 | 0 | 0 | 30.06 | 120.30 | 60.00 | 0.361 |
| 13 | 75.11* | 14.75 | 60.30 | 29.86 | 66.01 | 31.14 | 0.382 |
| 14 | 75.15* | 40.41 | 31.14 | 29.53 | 133.77 | 66.39 | 0.292 |
| 15 | 76.48 | 7.82 | 66.39 | 30.07 | 137.85 | 68.07 | 0.292 |
| 16 | 75.35 | 6.89 | 68.07 | 30.02 | 110.72 | 55.74 | 0.347 |
| 17 | 75.87 | 21.14 | 55.74 | 29.99 | 124.54 | 124.54 | 0.392 |

| | | | | | | | |
|---|---|---|---|---|---|---|---|
| * 1.25 wt percent PVP was used for these examples. | | | | | | | |

## Claims

1. A process for preparing crystalline particles of a drug substance comprising:
recirculating an anti-solvent through a mixing zone;
dissolving the drug substance in a solvent to form a solution;
adding the solution to the mixing zone to form a particle slurry in the anti-solvent;
**characterized in that**
at least a portion of the particle slurry is recirculated back through the mixing zone.

2. The process according to Claim 1 further comprising:
removing the solvent from the particle slurry to form a stripped slurry.

3. The process according to Claim 2 further comprising:
recycling a portion of the stripped slurry back through the mixing zone.

4. The process according to Claim 1 wherein the temperature of the mixing zone is from 0 to 65 °C.

5. The process according to Claim 1 wherein the solvent is selected from the group consisting of methanol, ethanol, isopropanol, 1-butanol, trifluoroethanol, polyhydric alcohols, amides, tetrahydrofuran, propionaldehyde, acetone, n-propylamine, isopropylamine, ethylene diamine, acetonitrile, methyl ethyl ketone, acetic acid, formic acid, dimethylsulfoxide, 1,3-dioxolane, hexafluoroisopropanol, and combinations thereof.

6. The process according to Claim 1 wherein the concentration of drug substance in the solution is from 0.1 to 20.0 weight percent.

7. The process according to Claim 1 wherein at least one stabilizer is added to the solution, to the anti-solvent or to both the solution and the anti-solvent.

8. The process according to Claim 7 wherein the stabilizer or stabilizers are polymeric stabilizers.

9. The process according to Claim 7 wherein the weight ratio of drug to total stabilizer in the particle slurry is from 0.1:1 to 10:1.

10. The process according to Claim 1 wherein the mixing zone comprises a mixer.

11. The process according to Claim 10 wherein the mixer is selected from the group consisting of a centrifugal pump, a recirculation loop, an in-line homogenizer, an ultrasonic mixer, an atomizer, a colloid mill and a combination thereof.

12. The process according to Claim 1 further comprising the step of removing the anti-solvent from at least a portion of the particle slurry.

13. The process according to Claim 12 wherein the anti-solvent is removed by way of spray drying, spray freezing, gellation, lyophilization, or filtration.

14. The process according to Claim 2 wherein the removing of the solvent comprises using membrane filtration, diafiltration, or evaporation.

15. The process according to Claim 14 wherein the removing of the solvent occurs using a wiped film evaporator.

16. The process according to Claim 2 further comprising the step of removing the anti-solvent from at least a portion of the stripped slurry.

17. The process according to Claim 16 wherein the anti-solvent is removed by way of spray drying, spray freezing, gellation, lyophilization, or filtration.

## Patentansprüche

1. Verfahren zur Herstellung kristalliner Partikel einer Arzneimittelsubstanz umfassend:
Rezirkulieren eines Anti-Lösungsmittels durch einen Mischungsbereich;
Lösen der Arzneimittelsubstanz in einem Lösungsmittel zur Bildung einer Lösung;
Zugeben der Lösung zu dem Mischungsbereich zur Bildung einer Partikelaufschlämmung in dem Anti-Lösungsmittel;
**dadurch gekennzeichnet,**
**dass** wenigstens ein Teil der Partikelaufschlämmung durch den Mischungsbereich zurück rezirkuliert wird.

2. Verfahren gemäß Anspruch 1 ferner umfassend Entfernen des Lösungsmittels aus der Partikelaufschlämmung zur Bildung einer ausgedämpften Aufschlämmung.

3. Verfahren gemäß Anspruch 2 ferner umfassend Wiederverwenden eines Teils der ausgedämpften Aufschlämmung durch den Mischungsbereich.

4. Verfahren gemäß Anspruch 1, wobei die Temperatur des Mischungsbereichs von 0 bis 65 °C reicht.

5. Verfahren gemäß Anspruch 1, wobei das Lösungsmittel ausgewählt ist aus der Gruppe bestehend aus Methanol, Ethanol, Isopropanol, 1-Butanol, Trifluorethanol, Polyalkoholen, Amiden, Tetrahydrofuran, Propionaldehyde, Aceton, n-Propylamin, Isopropylamin, Ethylendiamin, Acetonitril, Methylethylketon, Essigsäure, Ameisensäure, Dimethylsulfoxid, 1,3-Dioxolan, Hexafluorisopropanol und Kombinationen davon.

6. Verfahren gemäß Anspruch 1, wobei die Konzentration an Arzneimittelsubstanz in der Lösung von 0,1 bis 20,0 Gewichtsprozent reicht.

7. Verfahren gemäß Anspruch 1, wobei der Lösung, dem Anti-Lösungsmittel oder sowohl der Lösung als auch dem Anti-Lösungsmittel wenigstens ein Stabilisierungsmittel zugegeben wird.

8. Verfahren gemäß Anspruch 7, wobei das Stabilisierungsmittel oder die Stabilisierungsmittel polymere Stabilisierungsmittel sind.

9. Verfahren gemäß Anspruch 7, wobei das Gewichtsverhältnis von Arzneimittel zu Gesamtstabilisierungsmittel in der Partikelaufschlämmung von 0,1:1 bis 10:1 reicht.

10. Verfahren gemäß Anspruch 1, wobei der Mischungsbereich einen Mischer umfasst.

11. Verfahren gemäß Anspruch 10, wobei der Mischer ausgewählt ist aus der Gruppe bestehend aus einer Zentrifugalpumpe, einer Umwälzschleife, einem Inline-Homogenisator, einem Ultraschallmischer, einem Vernebler, einer Kolloidmühle und Kombinationen davon.

12. Verfahren gemäß Anspruch 1, ferner umfassend den Schritt Entfernen des Anti-Lösungsmittels aus wenigstens einem Teil der Partikelaufschlämmung.

13. Verfahren gemäß Anspruch 12, wobei das Anti-Lösungsmittel mittels Sprühtrocknung, Sprühgefrieren, Gelbildung, Lyophilisierung oder Filtration entfernt wird.

14. Verfahren gemäß Anspruch 2, wobei das Entfernen des Lösungsmittels eine Verwendung von Membranfiltration, Diafiltration oder Verdampfung umfasst.

15. Verfahren gemäß Anspruch 14, wobei die Entfernung des Lösungsmittels unter Verwendung eines Fraktionierbürstenverdampfers stattfindet.

16. Verfahren gemäß Anspruch 2 ferner umfassend den Schritt Entfernen des Anti-Lösungsmittels aus wenigstens einem Teil der abgedämpften Aufschlämmung.

17. Verfahren gemäß Anspruch 16, wobei das Anti-Lösungsmittel mittels Sprühtrocknung, Sprühgefrieren, Gelbildung, Lyophilisierung oder Filtration entfernt wird.

## Revendications

1. Procédé de préparation de particules cristallines d'une substance médicamenteuse comprenant :
le recyclage d'un anti-solvant à travers une zone de mélange ;
la dissolution de la substance médicamenteuse dans un solvant pour former une solution ;
l'ajout de la solution à la zone de mélange pour former une suspension épaisse de particules dans l'anti-solvant ;
**caractérisé en ce que**
au moins une partie de la suspension épaisse de particules est recyclée à travers la zone de mélange.

2. Procédé selon la revendication 1, comprenant en outre :
l'élimination du solvant de la suspension épaisse de particules pour former une suspension épaisse dépouillée.

3. Procédé selon la revendication 2, comprenant en outre :
le recyclage d'une partie de la suspension épaisse dépouillée à travers la zone de mélange.

4. Procédé selon la revendication 1, dans lequel la température de la zone de mélange est de 0 à 65 °C.

5. Procédé selon la revendication 1, dans lequel le solvant est choisi dans le groupe constitué par le méthanol, l'éthanol, l'isopropanol, le 1-butanol, le trifluoroéthanol, les polyols, les amides, le tétrahydrofuranne, le propionaldéhyde, l'acétone, la n-propylamine, l'isopropylamine, l'éthylènediamine, l'acétonitrile, la méthyléthylcétone, l'acide acétique, l'acide formique, le diméthylsulfoxyde, le 1,3-dioxolane, l'hexafluoroisopropanol, et les combinaisons de ceux-ci.

6. Procédé selon la revendication 1, dans lequel la concentration de la substance médicamenteuse dans la solution est de 0,1 à 20,0 pour cent en poids.

7. Procédé selon la revendication 1, dans lequel au moins un stabilisant est ajouté à la solution, à l'anti-solvant ou à la fois à la solution et à l'anti-solvant.

8. Procédé selon la revendication 7, dans lequel le stabilisant ou les stabilisants sont des stabilisants polymères.

9. Procédé selon la revendication 7, dans lequel le rapport en poids du médicament au stabilisant total dans la suspension épaisse de particules est de 0,1/1 à 10/1.

10. Procédé selon la revendication 1, dans lequel la zone de mélange comprend un mélangeur.

11. Procédé selon la revendication 10, dans lequel le mélangeur est choisi dans le groupe constitué par une pompe centrifuge, une boucle de recyclage, un homogénéisateur en ligne, un mélangeur à ultrasons, un atomiseur, une broyeuse colloïdale et une combinaison de ceux-ci.

12. Procédé selon la revendication 1, comprenant en outre l'étape consistant à éliminer l'anti-solvant d'au moins une partie de la suspension épaisse de particules.

13. Procédé selon la revendication 12, dans lequel l'anti-solvant est éliminé au moyen d'un séchage par pulvérisation, d'une congélation par pulvérisation, d'une gélification, d'une lyophilisation, ou d'une filtration.

14. Procédé selon la revendication 2, dans lequel l'élimination du solvant comprend l'utilisation d'une filtration à travers une membrane, d'une diafiltration, ou d'une évaporation.

15. Procédé selon la revendication 14, dans lequel l'élimination du solvant est effectuée en utilisant un évaporateur à couche balayée.

16. Procédé selon la revendication 2, comprenant en outre l'étape consistant à éliminer l'anti-solvant d'au moins une partie de la suspension épaisse dépouillée.

17. Procédé selon la revendication 16, dans lequel l'anti-solvant est éliminé au moyen d'un séchage par pulvérisation, d'une congélation par pulvérisation, d'une gélification, d'une lyophilisation, ou d'une filtration.
